# EUROPEAN PATENT SPECIFICATION

(11) **EP 1 274 384 B1**
(45) Date of publication and mention of the grant of the patent: **08.09.2004**
(21) Application number: 01920038.5
(22) Date of filing: 03.04.2001
(51) Int. Cl.: A61F 13/56, A61F 13/472

(54) **SANITARY NAPKIN**
DAMENBINDE
SERVIETTE HYGIENIQUE

(30) Priority: 13.04.2000 SE 0001378
(43) Date of publication of application: 15.01.2003
(73) Proprietor: SCA Hygiene Products AB, 405 03 Göteborg (SE)
(72) Inventor: HELMFRIDSSON, Bror-Inge, S-433 50 Partille (SE); DREVIK, Solgun, S-435 35 Mölnlycke (SE)
(74) Representative: Hyltner, Jan-Olof
(86) International application number: PCT/SE2001/000726
(87) International publication number: WO 2001/078638

(56) References cited:
- WO-A1-00/30585
- US-A- 5 453 143
- US-A- 5 454 804
- US-A- 5 713 886

## Description

### FIELD OF INVENTION

The present invention relates to an absorbent article, such as a sanitary napkin, a panty liner or an incontinence protector for women, that includes an absorption body sandwiched between a liquid-permeable and a liquid-impermeable outer sheet and having a front portion which is intended to face forwards when the article is worn, and a rear portion, wherein the absorbent body tapers rearwardly from a section of largest width situated in the front portion of the article to the rear portion thereof.

### BACKGROUND OF THE INVENTION

A sanitary napkin of this nature is intended to be worn in so-called string panties, which are very narrow at the rear portion of the crotch part of the napkin. It is also desirable to provide sanitary napkins that are intended for string panties with outwardly projecting wings or flaps that are intended to be folded around the edges of a panty and fastened to the outside thereof and/or to each other, since such wings have been widely accepted in respect of sanitary napkins that are intended for use in conventional panties or underpants.

Flaps that are intended to be folded around the edges of string panties are narrow of necessity, and are folded around fold lines that are inclined to the longitudinal symmetry axis of the napkin. Sanitary napkins are normally produced in continuous process lines with the napkin blanks moving with their longitudinal axes coincidental with the machine direction. The glue strings applied to the liquid-impermeable outer sheet and to the outsides of the flaps are normally applied with the aid of glue nozzles or similar devices and therefore extend in the machine direction . Because the flaps of sanitary napkins intended for string panties are narrow and are inclined to the machine direction, it is not possible to apply glue strings of sufficient sizes on these flaps in the same manner as glue strings are applied to the flaps of sanitary napkins that are intended for use with conventional underpants or panties.

It is known in certain contexts to apply glue strings that are inclined to the machine direction, with the aid of a so-called label principle. The label principle involves the use of a rotating roll that includes an oblique pick-up surface which collects glue from a glue bath and applies the glue to a travelling web of material. Such a device is expensive. The use of the label principle also places a limitation on the types of glue that can be used in a sanitary napkin manufacturing line, because certain types of glue are liable to foam in the glue bath at the production rates prevailing in such manufacture. Production speeds of about 250 m/min are not unusual in the manufacture of sanitary napkins.

In US 5,713,886 is disclosed a sanitary napkin to be worn in string panties having flaps that are inclined to the longitudinal symmetry axis of the diaper. One glue string is applied to each inclined flap and is inclined to the longitudinal symmetry axis of the napkin with the same inclination as the associate flap. Such a glue string can not be applied with the types of equipment normally used for glue application in manufacturing lines for sanitary napkins.

An object of the present invention is to solve the aforesaid problems associated with applying an adhesive to the flaps of a flap-equipped absorbent article in the form of a sanitary napkin, a panty liner or an incontinence protector for women intended for use in string panties.

### SUMMARY OF THE INVENTION

This object is achieved with an absorbent article in the form of a sanitary napkin, a panty liner or an incontinence protector for women that includes an absorbent body which is sandwiched between a liquid-permeable and a liquid-impermeable outer sheet and that has a front part which is intended to face forwards in use, and a rear part, wherein the absorbent body tapers rearwardly from a section of greatest width situated in the front part of the article to the end of the rear part of said article, and wherein the article is characterised in that it includes outwardly projecting, flexible flaps which extend beyond respective sides of the absorbent body along a part of the tapering portion of said body and which have longitudinal edges that are inclined relative to the longitudinal symmetry axis of the article at an angle such that the distance between the outer sides of the flaps and the longitudinal symmetry axis of the article decreases towards the rear end of said article; and in that at least two strings of adhesive are applied to at least one of the flaps on that side of said flap which, in the unfolded state of the flap, lies on the same side of the article as said liquid-impermeable outer sheet, said strings extending parallel with the longitudinal symmetry axis of the article and being offset in relation to each another both longitudinally and transversely, wherein when the article is flat each rearwardly lying string is located closer to the longitudinal symmetry line of the article than the forwardly lying string. By utilising several short longitudinally extending adhesive strings that are offset relative to one another both longitudinally and transversely instead of a continuous inclined adhesive string, it is possible to provide an "inclined adhesive string" with the same type of equipment as that normally used in sanitary napkin manufacturing lines.

In one preferred embodiment of the invention, the flaps are integral parts of the outer sheets and at least one adhesive string is applied to that part of a napkin that includes the absorbent body. The adhesive used will preferably be a pressure-sensitive hotmelt glue. The adhesive strings applied to the flaps may also mutually overlap longitudinally. The adhesive strings applied to the flaps are preferably rectangular in shape and of mutually the same size, and an imaginary line that passes through the transversely innermost corners of the strings on one and the same flap will have the same angle of inclination to the longitudinal symmetry axis of the article as the outer longitudinal edge of said flap.

### BRIEF DESCRIPTION OF THE DRAWING

The invention will now be described with reference to the accompanying drawing, in which
Fig. 1 is a schematic partially sectioned plan view of a first embodiment of an inventive sanitary napkin; and
Fig. 2 is a schematic plan view of a second embodiment of an inventive sanitary napkin.

### DESCRIPTION OF EMBODIMENTS

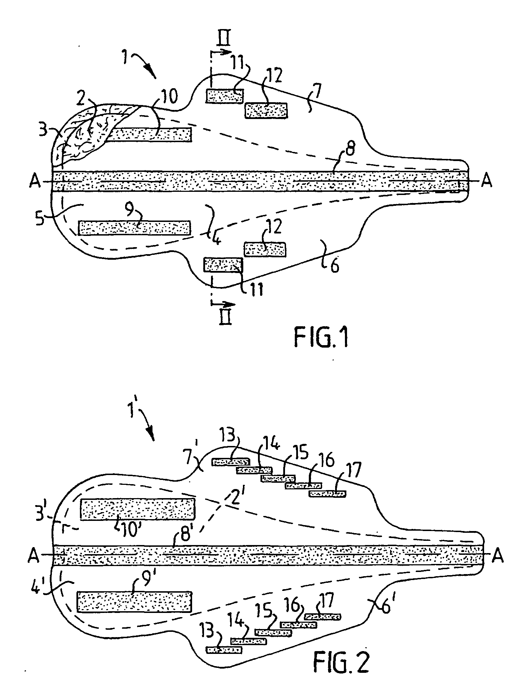

The sanitary napkin 1 shown in Figure 1 typically includes an absorption body 2 that is enclosed between a liquid-permeable outer sheet 3 and a liquid-impermeable outer sheet or backing sheet 4. The outer sheets 3, 4 are mutually joined at parts which lie outside the absorbent body in some suitable way, e.g. by gluing or by ultrasound welding or heat welding.

The napkin 1 is intended for use in a string panty and the absorbent body 2 therefore tapers rearwardly from the widest part of a front portion 5 of the napkin to the rear end thereof. The outer sheets 3, 4 form outwardly projecting flaps 6, 7 or wings that are intended to be folded around the edges of a string panty and fastened to the outside thereof. The flaps 6, 7 extend longitudinally outside the edges of the absorbent body with essentially the same width, and the longitudinal edges of the flaps therewith converge towards each other in the rearward direction. In the illustrated embodiment, the length of the flaps corresponds to about half the length of the napkin and the flaps are spaced further from the front end of the napkin than from its rear end.

The illustrated napkin has a length of 140-260 mm. The absorbent body has a largest width of 70 mm and a smallest width of about 10 mm at its rear end. The flaps extend through a distance of about 25-30 mm beyond the edges of the absorbent body with the greatest distance at the forward portions of the flaps. The front edges of the flaps are located about 60 mm from the front edge of the napkin, and their rear edges are situated about 50 mm from the rear end of said napkin. It will be understood that these measurements are only intended to give a qualitative understanding of a suitable design of a sanitary napkin intended for string panties and in no way limit the scope of the invention.

The liquid-permeable outer sheet 3 is comprised of a soft skin-friendly material. This outer sheet may comprise different types of nonwoven fibre material. Other materials that can be used include perforated plastic films, plastic nets, knitted, crocheted or woven fabrics and combinations and laminations of said materials. The plastic may be a thermoplastic, e.g. polyethylene (PE). The nonwoven material may comprise natural fibres, such as cellulose or cotton, although it may alternatively comprise synthetic fibres, such as polyethylene (PE), polypropylene (PP), polyurethane (PU), a polyester, nylon or regenerated cellulose, or a mixture of different fibres. All materials used in the manufacture of liquid-permeable outer sheets for absorbent articles, such as sanitary napkins, panty liners or incontinence protectors can be used for the liquid-impermeable outer sheet 3 and it will be understood that the aforesaid material is given solely by way of example.

The liquid-impermeable outer sheet 4 is comprised of flexible material, preferably a thin plastic film of polyethylene (PE), polypropylene (PP) or a polyester, although it may consist of liquid-permeable material, such as nonwoven material, laminated with a liquid-impermeable material. All materials used to produce liquid-impermeable outer sheets for absorbent articles can be used. The outer sheet 4 may beneficially be air-permeable.

The absorbent body 2 is preferably composed from cellulose fibres, although other natural materials, such as cotton fibres or peat can be used. Alternatively, absorbent synthetic fibres or a mixture of natural fibres and synthetic fibres may be used. The absorbent body 2 may also include a superabsorbent, i.e. a polymer that is able to absorb liquid in an amount corresponding to several times its own weight. The absorbent body may also include shape stabilising means and liquid dispersing means, and also a binder which functions to hold short fibres and particles together in a coherent unit. The absorbent may also be comprised of more than one layer of absorbent material.

Although the flaps 6, 7 of the illustrated embodiment are comprised of laterally extended portions of the outer sheets 3, 4, they may comprise extended portions of solely one of said sheets. The flaps may also comprise separate pieces of material fastened to the sides of the napkin 1.

The liquid-impermeable outer sheet or backing sheet 4 of the napkin 1 is provided with three adhesive strings 8, 9, 10 in the region of the absorbent body 2, these being a central adhesive string 8 that extends along the longitudinal symmetry axis A-A of the napkin over the full length thereof, and two shorter adhesive strings 9, 10 that extend on respective sides of the central adhesive string 8 on the front part 5 of said napkin. These adhesive strings 8, 9, 10 are intended to fasten the napkin 1 to the inside of a string panty. Each of the adhesive strings 8, 9, 10 extend parallel with the longitudinal symmetry axis A-A of the napkin 1.

The napkin 1 is also provided with two adhesive strings 11, 12 on each flap 6, 7. These adhesive strings 11, 12 are relatively short and extend parallel with the longitudinal symmetry axis A-A of the napkin when the napkin is flat. The adhesive strings 11, 12 on each flap 6; 7 are also mutually spaced both longitudinally and laterally, wherewith the foremost string 11 is situated furthest from the longitudinal symmetry axis A-A. Each adhesive string 11, 12 will conveniently have a length shorter than about 30 mm, and preferably a length within the range of 10-20 mm. Each adhesive string will conveniently have a width of between 2-15 mm.

Although only two adhesive strings 11, 12 are disposed on each flap 6, 7 of the Figure 1 embodiment, it will be understood that the flaps may be provided with more adhesive strings if so desired.

Figure 2 illustrates a second embodiment of the invention that differs from the embodiment shown in Figure 1 solely by the fact that the flaps 6', 7' are provided with an adhesive string pattern that is different to the pattern on the flaps 6, 7 of the Figure 1 embodiment. Those components of the sanitary napkin 1' shown in Figure 2 that find correspondence in similar components of the napkin 1 shown in Figure 1 have been identified with the same reference sign to which a prime has been added. As opposed to the pattern of adhesive strings applied to the flaps 6, 7, which patterns do not extend over the rear portions of the flaps, the pattern of adhesive strings 13, 17 on the flaps 6', 7' extend longitudinally over the major part of said flaps. Furthermore, the adhesive strings 14-17 overlap forwardly lying adhesive strings in the longitudinal direction. Thus, the mutually overlapping short adhesive strings form an essentially continuous adhesive string that is inclined to the longitudinal symmetry axis A-A. The adhesive strings applied to the flaps are generally rectangular in shape and an imaginary line passing through the transversely innermost corners of the strings on one and the same flap is inclined to longitudinal symmetry axis of the article at the same angle as the outer longitudinal edge of said flap. The rectangular adhesive strings applied to the flaps will preferably have mutually the same size.

The adhesive in the adhesive strings is a pressure-sensitive hotmelt glue, e.g. Ecomelt H145 from Collano, Switzerland, although other commercially available pressure-sensitive adhesive can be used, including adhesives that are pressure-sensitive in a cold state, such as acrylate glue normally combined with stickiness-enhancing additives, such as polyterpene, or hotmelt glue such as styrene and butadiene co-polymers.

In the packaged state of the sanitary napkins 1, 1', the adhesive strings are covered by a protective layer, e.g. a layer of release paper that consists of silicone-coated paper and protects the adhesive strings against contaminants, such as dust and similar substances, and also prevents the glue from drying out prior to use. The napkin 1, 1' is conveniently provided with a central protective layer that is not removed until the time when the napkin shall be fastened to the inside of a string panty, and each flap is provided with a protective layer that is removed prior to folding the flap around the edge of a string panty and fastening said flap to the outside thereof. These protective layers or backings are not shown in the Figures for the sake of clarity.

The sanitary napkins 1, 1' are manufactured in so-called length production in a continuous production line, by which is meant that the machine direction coincides with the longitudinal axis of the napkin blanks. In production, absorbent bodies are placed on one travelling web of outer sheet material, whereafter the other web of outer sheet material is placed on the web that is comprised of the first outer sheet and the absorbent bodies. The adhesive strings and the protective layers may either be applied subsequent to having delivered outer sheeting and absorbent bodies to the production line, or may be applied to the liquid-impermeable outer sheet prior to combining said sheet with the absorbent bodies and the liquid-permeable outer sheet. Individual napkins are cut from the resultant web of napkin blanks in the final stage of manufacture.

The adhesive strings can either be applied to the protective layers prior to applying said layers to the liquid-impermeable outer sheet, or may be applied to the liquid-impermeable outer sheet prior to applying the protective layers.

The sanitary napkins 1, 1' or the liquid-impermeable outer sheet will thus have the flat state shown in the Figures when applying the adhesive strings.

Because the adhesive strings applied to the liquid-impermeable outer sheet all have a linear extension in the machine direction of the production line, the strings can be applied with the aid of simple glue nozzles or similar devices. Furthermore, the aforedescribed principle of creating generally continuous adhesive strings that extend in a direction which is inclined to the machine direction, by compiling the "continuous" adhesive strings from a number of short adhesive strings that are offset relative to each other both longitudinally and transversely, enables sanitary napkins that are provided with flaps and intended for wear in string panties to be provided with one or more continuous adhesive strings that are inclined relative to the machine direction on each flap without needing to use complicated glue applicating equipment and without the glue applying process detracting from the rate of production or the choice of adhesive.

It will be understood that the described embodiments can be modified within the scope of the invention. For instance, the absorbent body may include a central outwardly projecting part and the sanitary napkin may have dimensions other than those aforedescribed. The glue pattern on the part of the napkin that includes the absorbent body may also be different to that described, for instance the central adhesive string need not extend in the napkin over the whole of the front portion thereof, and the three adhesive strings disposed in the front portion of the napkin may be replaced with a single glue string that extends over the major part of the front portion of the napkin. More than three glue strings may be provided on the part of the napkin that includes the absorbent body. It is also conceivable to replace the adhesive strings in the region of the absorbent body with friction coatings or similar means. Moreover, adhesive strings may be provided solely on one flap, in which case it is necessary to dimension the flaps so that they overlap each other when folded in towards the underside of the string panty subsequent to having been folded around the edges thereof. The invention is therefore restricted solely by the contents of the accompanying Claims.

## Claims

1. An absorbent article (1; 1') in the form of a sanitary napkin, a panty liner or an incontinence protector for women, comprising an absorbent body (2; 2') that is enclosed between a liquid-permeable outer sheet (3; 3') and a liquid-impermeable outer sheet (4; 4'), wherein said article has a front part (5) which is intended to face forwards when the article is worn, and a rear part, and wherein the absorbent body tapers rearwardly to the end of the rear diaper part from a section of greatest width situated in said front part, **characterised in that** the article (1; 1') includes flexible outwardly projecting flaps (6, 7; 6', 7') which extend on respective sides of the absorbent body (2; 2') along a part of the tapering portion of the absorbent body and have outer longitudinally extending edges that are inclined relative to the longitudinal symmetry axis (A-A) at an angle such that the distance between the outer sides of said flaps and the longitudinal symmetry axis of the article decreases in a direction towards the rear end of the article; and **in that** at least two adhesive strings (11, 12; 13-17) are applied to at least one of the flaps on the side thereof which, in the unfolded state of the flap, lies on the same side of the article as the liquid-impermeable outer sheet, wherein said strings extend parallel with the longitudinal symmetry axis of the article and are offset relative to each other both longitudinally and transversely, and wherein when the article is flat each rearwardly lying string is situated closer to the longitudinal symmetry line of the article than the forwardly lying string.

2. An absorbent article (1; 1') according to Claim 1, **characterised in that** the flaps (6, 7; 6', 7') are integral with the outer sheets.

3. An absorbent article (1; 1' ) according to Claim 1 or 2, **characterised in that** the part of the sanitary napkin (1; 1') that includes the absorbent body (2; 2') has at least one adhesive string (8-10; 8'-10').

4. An absorbent article (1; 1') according to Claim 3, **characterised in that** the adhesive strings (11, 12; 13-17) consist of pressure-sensitive glue.

5. An absorbent article (1; 1') according to Claim 4, **characterised in that** the pressure-sensitive glue is a hotmelt glue.

6. An absorbent article (1; 1') according to any one of Claims 1-5, **characterised in that** the adhesive strings (11, 12; 13-17) applied to the flaps (6, 7; 6', 7') overlap each other in the longitudinal direction.

7. An absorbent article (1; 1') according to Claim 6, **characterised in that** the adhesive strings (13-17) on the flaps (6', 7') are rectangular in shape; and **in that** an imaginary line passing through the transversely innermost corners of the strings on one and the same flap are inclined to the longitudinal symmetry axis (A-A) of the article at the same angle as the outer longitudinal edge of said flap.

8. An absorbent article (1; 1') according to Claim 7, **characterised in that** the rectangular adhesive strings (13-17) on the flaps (6', 7') are mutually of the same size.

## Patentansprüche

1. Absorbierender Gegenstand (1; 1') in der Form einer Binde, einer Slipeinlage oder einem Inkontinenzschutz für Damen, umfassend einen Absorptionskörper (2; 2'), der zwischen einer flüssigkeitsdurchlässigen Oberschicht (3; 3') und einer flüssigkeitsundurchlässigen Außenschicht (4; 4') eingeschlossen ist, wobei der Gegenstand einen Vorderteil (5), der dazu gedacht ist, nach vorne zu weisen, wenn der Gegenstand getragen wird und einen Hinterteil aufweist und wobei sich der Absorptionskörper von einem Abschnitt mit einer größten Breite, der in dem Vorderteil angeordnet ist, zu dem Ende des Hinterteils nach hinten verjüngt,
**dadurch gekennzeichnet, dass**
der Gegenstand (1; 1') flexible nach außen vorragende Flügel (6, 7; 6', 7') aufweist, die sich auf entsprechenden Seiten des Absorptionskörpers (2; 2') entlang eines Teils des verjüngten Abschnitts des Absorptionskörpers erstrecken und die äußere sich längs erstreckende Kanten aufweisen, die relativ zu der Längssymmetrieachse (A-A) in einem Winkel geneigt sind, so dass der Abstand zwischen den äußeren Seiten der Flügel und der Längssymmetrieachse des Gegenstands in einer Richtung zu dem Hinterende des Gegenstands vermindert ist; und dadurch, dass wenigstens zwei Klebestreifen (11, 12; 13-17) auf wenigstens einem der Flügel auf der Seite davon aufgebracht sind, die in dem ungefalteten Zustand des Flügels auf der gleichen Seite des Gegenstandes wie die flüssigkeitsundurchlässige Außenschicht liegt, wobei sich die Streifen parallel zu der Längssymmetrieachse des Gegenstands erstrecken und relativ zueinander sowohl längs als auch quer versetzt sind und wobei wenn der Gegenstand flach ist, jeder hinten liegende Streifen näher zu der Längssymmetrieachse des Gegenstandes angeordnet ist als der davor liegende Streifen.

2. Absorbierender Gegenstand (1; 1') nach Anspruch 1, **dadurch gekennzeichnet, dass** die Flügel (6, 7; 6', 7') integral mit den Außenschichten ausgebildet sind.

3. Absorbierender Gegenstand (1; 1') nach Anspruch 1 oder 2, **dadurch gekennzeichnet, dass** der Teil der Damenbinde (1; 1'), der den Absorptionskörper (2; 2') aufweist, wenigstens einen Klebestreifen (8-10; 8'-10') aufweist.

4. Absorbierender Gegenstand (1; 1') nach Anspruch 3, **dadurch gekennzeichnet, dass** die Klebestreifen (11, 12; 13-17) aus einem Haftklebstoff bestehen.

5. Absorbierender Gegenstand (1; 1') nach Anspruch 4, **dadurch gekennzeichnet, dass** der Haftklebstoff ein Schmelzklebstoff ist.

6. Absorbierender Gegenstand (1; 1') nach einem der Ansprüche 1-5, **dadurch gekennzeichnet, dass** die Klebestreifen (11, 12; 13-17), die auf den Flügeln (6, 7; 6', 7') aufgebracht sind, einander in der Längsrichtung überlappen.

7. Absorbierender Gegenstand (1; 1') nach Anspruch 6, **dadurch gekennzeichnet, dass** die Klebestreifen (13-17) auf den Flügeln (6', 7') in ihrer Form rechteckig sind; und dadurch, dass eine imaginäre Linie, die durch die quer gesehen innersten Ecken der Streifen auf ein und demselben Flügel verläuft, zu der Längssymmetrieachse (A-A) des Gegenstands in dem gleichen Winkel wie die äußeren Längskanten des Flügels geneigt ist.

8. Absorbierender Gegenstand (1; 1') nach Anspruch 7, **dadurch gekennzeichnet, dass** die rechteckigen Klebestreifen (13-17) auf den Flügeln (6', 7') zueinander die gleiche Größe aufweisen.

## Revendications

1. Un article absorbant (1; 1') en forme de serviette hygiénique, de protège-dessous ou de protection féminine d'incontinence, comportant un corps absorbant (2; 2') qui est enfermé entre une feuille extérieure perméable aux liquides (3; 3') et une feuille extérieure imperméable aux liquides (4; 4'), lequel dit article présente une partie frontale (5) qui est conçue pour être orientée vers l'avant lorsque l'article est porté, et une partie arrière, et dans lequel le corps absorbant s'effile vers l'arrière jusqu'à l'extrémité de la partie arrière de la couche, à partir d'une section de largeur maximale située dans ladite partie frontale, **caractérisé en ce que** l'article (1; 1') comprend des ailes flexibles faisant saillie vers l'extérieur (6, 7; 6', 7') qui s'étendent sur les côtés respectifs du corps absorbant (2; 2') le long d'une partie de la portion effilée du corps absorbant et présentent des bords extérieurs s'étendant longitudinalement qui sont inclinés par rapport à l'axe de symétrie longitudinal (A-A) à un angle tel que la distance entre les côtés extérieurs desdites ailes et l'axe de symétrie longitudinal de l'article diminue en direction de l'extrémité arrière de l'article; et **en ce qu'**au moins deux bandes adhésives (11, 12; 13-17) sont appliquées à au moins une des ailes sur son côté qui, lorsque l'aile est à l'état déplié, se trouve sur le même côté de l'article que la feuille extérieure imperméable aux liquides, dans lequel lesdites bandes s'étendent parallèlement avec l'axe de symétrie longitudinal de l'article et sont décalées l'une par rapport à l'autre aussi bien longitudinalement que transversalement, et dans lequel lorsque l'article est plat chaque bande se trouvant à l'arrière est située plus près de la ligne de symétrie longitudinale de l'article que la bande se trouvant à l'avant.

2. Un article absorbant (1; 1') selon la revendication 1, **caractérisé en ce que** les ailes (6, 7; 6', 7') font partie intégrante avec les feuilles extérieures.

3. Un article absorbant (1; 1') selon la revendication 1 ou 2, **caractérisé en ce que** la partie de la serviette hygiénique (1; 1') qui comprend le corps absorbant (2; 2') présente au moins une bande adhésive (8-10; 8'-10').

4. Un article absorbant (1; 1') selon la revendication 3, **caractérisé en ce que** les bandes adhésives (11, 12; 13-17) se composent de colle sensible à la pression.

5. Un article absorbant (1; 1') selon la revendication 4, **caractérisé en ce que** la colle sensible à la pression est une colle thermofusible.

6. Un article absorbant (1; 1') selon l'une quelconque des revendication 1-5, **caractérisé en ce que** les bandes adhésives (11, 12; 13-17) appliquées sur les ailes (6, 7; 6' 7') se chevauchent l'une l'autre dans la direction longitudinale.

7. Un article absorbant (1; 1') selon la revendication 6, **caractérisé en ce que** les bandes adhésives (13-17) sur les ailes (6', 7') sont de forme rectangulaire; et **en ce qu'**une ligne imaginaire passant à travers les coins les plus à l'intérieur des bandes transversalement sur une et la même aile est inclinée par rapport à l'axe de symmétrie longitudinal (A-A) de l'article au même angle que le bord longitudinal extérieur de ladite aile.

8. Un article absorbant (1; 1') selon la revendication 7, **caractérisé en ce que** les bandes adhésives rectangulaires (13-17) sur les ailes (6' 7') sont mutuellement de la même grandeur.
